# EUROPEAN PATENT SPECIFICATION

(11) **EP 0 650 734 B1**
(45) Date of publication and mention of the grant of the patent: **10.03.1999**
(21) Application number: 93117518.6
(22) Date of filing: 28.10.1993
(51) Int. Cl.: A61K 39/295, A61K 39/12

(54) **Pentavalent live viral vaccine**
Pentavalenter, viraler Lebendimpfstoff
Vaccin viral vivant pentavalent

(43) Date of publication of application: 03.05.1995
(73) Proprietor: DIVISION OF MICROBIOLOGY, KYOTO BIKEN LABORATORIES, INC., Uji-shi, Kyoto 611 (JP)
(72) Inventor: Fukuyama, Shin-ichi, Sourakugun, Kyoto (JP); Kodama, Kazuo, Uji-shi, Kyoto (JP); Takamura, Keizou, Uji-shi, Kyoto (JP); Shirakawa, Yumi, Mitaka-shi Tokyo 181 (JP); Kawazu, Kentarou, Uji-shi, Kyoto (JP); Izumida, Akihiro, Tsuzuki-gun, Kyoto (JP); Ishida, Yasuhisa, Kyoto-shi, Kyoto (JP)
(74) Representative: VOSSIUS & PARTNER

(56) References cited:
- EP-A- 0 252 059
- US-A- 3 634 587
- BIOLOGICAL ABSTRACTS, xol. 79, no. 8 1985, Philadelphia, PA, US; abstract no. 68009, J.C. FRENNET ET AL. 'SAFETY AND EFFICACY OF A COMBINED LIVE BOVINE RESPIRATORY SYNCYTIAL VIRUS AND BOVINE VIRAL DIARRHEA VACCINE FOR REARING CALVES.' page AB-437 ; & ANN. MED.VET. vol. 128, no. 5 , 1984 pages 375 - 383
- PATENT ABSTRACTS OF JAPAN vol. 10, no. 235 (C-366)(2291) 14 August 1986 & JP-A-61 068 428 (NIPPON SEIBUTSU KAGAKU KENKYUSHO) 8 April 1986
- DATABASE CAB CAB INTERNATIONAL, WALLINGFORD, OXON, GB 81:48588 H. ANTOINE 'RESPIRATORY DISEASES OF CATTLE IN BELGIUM.' & BULLETIN DES GROUPEMENTS TECHNIQUES VETERINAIRES no. 5 , 1980 pages 35 - 44
- FENNER ET AL. 'Veterinary Virology', 1987, HARCOURT BRACE JOVANOVICH, ACADEMIC PRESS, INC.

## Description

A polyvalent live vaccine for infectious bovine rhinotracheitis virus infection, bovine viral diarrhea-mucosal disease virus infection, parainfluenza 3 virus infection, bovine adeno 7 virus infection.

The present invention relates to a polyvalent live vaccine which combines the vaccines for each of the following virus infections: infectious bovine rhinotracheitis (IBR) virus infection, bovine viral diarrhea-mucosal disease (BVD-MD) virus infection, parainfluenza 3 (PI3) virus infection, bovine RS virus infection and bovine adeno 7 (AD7) virus infection.

Infection by the infectious bovine rhinotracheitis (IBR) virus causes respiratory symptoms, conjunctivitis and vulvitis, etc. in cattle and abortion for pregnant cows. The bovine viral diarrhea-mucosal disease (BVD-MD) virus is known to cause, in addition to respiratory symptoms, seriously fatal diarrhea, and is a cause of persistent infection of fetus or calf when pregnant cows are infected, as well as abnormalities of gestation such as abortion. The parainfluenza 3 (PI3) virus is a primary cause of respiratory symptoms. The bovine RS virus also causes primarily respiratory disease, and symptoms usually so severe and long-lasting that in adult cattle, sometimes death was observed. Further, infection by the bovine adeno 7 (AD7) virus is a cause of both respiratory symptoms and diarrhea.

These five types of pathogens are widely spread among herds of cattle in Japan and other countries, causing repeated infections and outbreaks of the diseases. They therefore constitute a constant threat to cattle in various stages of growth, as well as in full grown cattle, as such outbreaks lead to death, retarded growth, reduction in milk secretion, reduction in calf production, etc., increasing the total damage given to the cattle industry. It is here that the vaccines for these five types of viruses are most useful, and that a polyvalent live vaccine would be most profitable economically.

Each of the individual vaccines were developed with an object to prevent these five types of diseases individually, and have been utilized to date. In addition, utilization has been made in recent years of a trivalent live vaccine, which includes the vaccines for infectious bovine rhinotracheitis (IBR) virus infection, bovine viral diarrhea-mucosal disease (BVD-MD) virus infection, and parainfluenza 3 (PI3) virus infection (see US patent No. 3,634,587). Nevertheless, no mixture has been developed which contains all 5 of the above mentioned vaccines in sufficient concentration to be effective. Five times the concentration of viruses contained in a single vaccine is required to mix the five types of vaccine viruses, and it has been impossible according to existing methods to obtain a vaccine with five times the total amount of viruses of a single vaccine. A mixture of all the vaccine viruses in a single, polyvalent vaccine has thus been impossible until now.

The present invention was developed in light of the above circumstances, by adding a vaccine virus solution for bovine RS virus infection and bovine adeno 7 (AD7) virus infection to the above mentioned trivalent live vaccine in order to overcome the disadvantages associated with mixtures of vaccine viruses described above.

That is, the present invention is a polyvalent live vaccine composed of a mixture of 5 types of attenuated virus suspensions, namely, a virus suspension prepared by culturing attenuated infectious bovine rhinotracheitis (IBR) virus in bovine or swine tissue cultures; a virus suspension prepared by culturing attenuated bovine viral diarrhea-mucosal disease (BVD-MD) virus in bovine or swine tissue cultures; a virus suspension prepared by culturing attenuated parainfluenza 3 (PI3) virus in chicken or bovine tissue cultures, or monkey kidney (vero) cells; a virus suspension prepared by culturing attenuated bovine RS virus in hamster or bovine tissue cultures, or monkey kidney (vero) cells; and a virus suspension prepared by culturing attenuated bovine adeno 7 (AD7) virus in goat or bovine tissue cultures.

For the above mentioned attenuated infectious bovine rhinotracheitis (IBR) virus suspension, the amount of viruses to be inoculated is set to a multiplicity of infection (amount of inoculated virus/number of cells grown; hereunder abbreviated as "m.o.i.") of 0.001 and culturing is done for 12-14 days, making it possible to provide a virus fluid which is about 5-7 times as concentrated as one obtained according to conventional methods.

For the attenuated bovine viral diarrhea-mucosal disease (BVD-MD) virus suspension the amount of inoculation is modified for an m.o.i. of 0.5-0.1, and by mixed cultivation of this amount of inoculation virus and a cell suspension at 34°C, it is possible to provide a virus fluid which is about 5-10 times as concentrated as one obtained according to conventional methods.

Also, for the attenuated parainfluenza 3 (PI3) virus suspension, by mixed cultivation of a cell suspension and the virus, it is possible to provide a virus fluid which is 5-8 times as concentrated as one obtained according to conventional methods.

Further, for the attenuated bovine RS virus suspension, a method was developed and applied wherein the best cells for virus growth were selected by cloning from the HAL cells, and the collected virus fluid was further subjected to ultrafiltration in order to increase the concentration thereof by about 5-10 times. A virus fluid thus prepared is 5-8 times as concentrated as one obtained according to conventional methods.

In addition, for the attenuated bovine adeno 7 (AD7) virus suspension, cultivation was done with an m.o.i. of 0.1, making it possible to provide a virus fluid which is 5-8 times as concentrated as one obtained according to conventional methods.

As described above, the concentration of virus in each single vaccine is increased to 5 times or more in each of the virus fluids, thus making it possible to develop a polyvalent live vaccine.

The mixture ratio for each of the vaccine viruses was 10^{7.75}TCID₅₀ (TCID₅₀ stands for Median Tissue Culture Infection Dose; Fenner, F., Bachmann, P.A., Gibbs, E.P.J., Murphy, F.A., Studdert, M.J., and White, D.O., Veterinary Virology, Harcourt, Brace, Jovanovich (eds.), Academic Press, Inc. (1987), pp.49-50) or over for the infectious bovine rhinotracheitis (IBR) virus; 10^{6.00}TCID₅₀ or over for the bovine viral diarrhea-mucosal disease (BVD-MD) virus; 10^{8.00}TCID₅₀ or over for the parainfluenza 3 (PI3) virus; 10^{7.75}TCID₅₀ or over for the bovine RS virus; and 10^{6.75}TCID₅₀ or over for the bovine adeno 7 (AD7) virus. The polyvalent live vaccine was thus obtained as a product with stable effectiveness in fighting infection of each virus.

In addition, a stabilizer (an aqueous solution containing 5% by weight JIS (JIS stands for Japanese Industrial Standard) reagent special grade lactose, 5% by weight JIS reagent special grade sucrose, 2% by weight JIS reagent special grade L-arginine hydrochloride, 3% by weight JIS reagent special grade glycine, 0.3% by weight JIS reagent special grade polyvinylpyrrolidone, 0.5% by weight JIS reagent special grade lactalbumin and 0.1% by weight JIS reagent special grade yeast extract) was developed in order to preserve the stability of the product during and after lyophilization and thus prevent inactivation of the 5 types of attenuated viruses in the mixture.

The present invention is a polyvalent live vaccine prepared by mixing an attenuated infectious bovine rhinotracheitis (IBR) virus, attenuated bovine viral diarrhea-mucosal disease (BVD-MD) virus, attenuated parainfluenza 3 (PI3) virus, attenuated bovine RS virus and attenuated bovine adeno 7 (AD7) virus, which may be utilized as a single product. There is no example in the world where such a polyvalent live vaccine is utilized.

Also, a polyvalent live vaccine according to the present invention gives the following excellent effects:
(1) These five types of pathogens are prevalent year round, and the transmission route of infection is by contact of individual cattle, whereby they have invaded practically all herds of cattle throughout every part of the world. Thus, this polyvalent live vaccine is needed for prevention of the five types of pathogens in the cattle industry.
(2) The same effects may be realized with only a single inoculation of the polivalent vaccine, as opposed to separate inoculations with each of the five kinds of vaccines.
(3) There are no observed interfering phenomena where the effects of these five live vaccines are inhibited, in either in vitro tests or in vivo tests using cattle.
(4) Even when this polyvalent live vaccine is inoculated into cattle, they show no clinical symptoms such as depression or anorexia, nor any abnormalities in red blood cell or white blood cell counts. Also, there is no swelling or induration at the location of injection. Further, there is no pyrexia (40.5°C or over) or leukopenia.
(5) Since effects against 5 types of diseases are obtainable with a single vaccination, it is possible to reduce the number of times of holding cattle and the number of times of injection, providing a reduction in stress undergone by the individual cattle. This also allows for a decreased number of staff stationed to provide the injections, as well as the number of their working hours, and is therefore more economical.
(6) Since the polyvalent live vaccine is a single product, the vaccine production cost is reduced to a cost which is lower the sum of producing each of the vaccines individually, and is therefore more economical for stockbreeding farmers.

### 4. Working Examples

A method of producing a polyvalent live vaccine against infectious bovine rhinotracheitis (IBR) virus infection, bovine viral diarrhea-mucosal disease (BVD-MD) virus infection, parainfluenza 3 (PI3) virus infection, bovine RS virus infection and bovine adeno 7 (AD7) virus infection according to the present invention is described below.

First, for the attenuated infectious bovine rhinotracheitis (IBR) virus, wild-type highly pathogenic No. 758 strain was passaged for 39 generations in swine testicle cell cultures at 30°C, and the No. 758-43 strain resulting from cloning three times by limiting dilution was used as the seed virus. Cloning is a method which is generally practiced in this field of industry. A brief explanation of cloning by limiting dilution is given hereunder. Virus fluid (a subject) is diluted to such a concentration as to include, for example, one virus in a fluid of 0.1*m*ℓ. The virus then increases in cells to form a uniform group, which is called a clone virus, and the operation as described above is cloning. Here, cloning is done three times. This is to make it secure to obtain the clone virus. This seed virus was inoculated into swine testicle cell cultures which had been cultured at 37 °C for 2-4 days, at a m.o.i. of 0.001, and the virus fluid which was collected after culturing at 30°C for 12-14 days was used as a virus suspension of the attenuated infectious bovine rhinotracheitis (IBR) virus.

Next, for the attenuated infectious bovine viral diarrhea-mucosal disease (BVD-MD) virus, wild-type highly pathogenic No. 12 strain was passaged in swine testicle cell cultures at 34 °C for 39 generations, and the No. 12-43 strain resulting from cloning three times by limiting dilution was used as the seed virus. This seed virus was inoculated into the cell suspension of a swine testicle at a concentration of 1 million cells per milliliter at a m.o.i. of 0.5-0.1, and the virus fluid which was obtained after culturing at 34°C for 3 days was used as a virus suspension of the attenuated bovine viral diarrhea-mucosal disease (BVD-MD) virus.

Also, for the attenuated parainfluenza 3 (PI3) virus, wild-type highly pathogenic BN₁-1 strain was serially passaged in a culture of chicken embryo cells at 30°C for 15 generations, and the resulting BN-CE strain was used as the seed virus. This seed virus was inoculated into chicken embryo cell suspensions at a concentration of 3 million cells per milliliter at a m.o.i. of 0.01, and the virus fluid which was collected after culturing at 30°C for 8-10 days was used as a virus suspension of the attenuated parainfluenza 3 (PI3) virus.

Further, for the attenuated bovine RS virus, wild-type highly pathogenic RS-52 strain was serially passaged in HAL cells at 34 °C for 5 generations and at 30°C for 10 generations, after which cloning was done three times by limiting dilution, and the resulting temperature sensitive mutant was used as the seed virus. This seed virus was inoculated into highly virus-sensitive HAL cell cultures obtained by further cloning at a m.o.i. of 0.01, and a virus fluid was collected after culturing at 30 °C for 7-10 days. Then, the virus solution whose concentration was increased by 5-10 times using fractional ultrafiltration was used as a virus suspension of the attenuated bovine RS virus.

The following is a description about HAL cell used for the attennuation of the bovine RS virus. While passaging and incubating an adult hamster lung culture cell, it was observed that a colony of cells with unusual form appeared in the 7th generation. The cell obtained from this colony was passaged for 20 generations, followed by three times clonings. After passaging two more generations, the cell of 25th generation was obtained to be used as a master seed virus, which was freezed in a liquid nitrogen for preservation. The cell which is younger than 20th generation from the master seed virus is used for preparation. The properties of this HAL cell is as follows: Its appearance is an epithelial cell. No microorganism is observed to stray thereinto. This HAL cell can easily be propagated within a glassware. Serial passage test records that this cell was passaged for up to 150 generations. This is a strain cell and can be incubated by permanent passages. In sensibility tests for virus, Ibaraki virus, bovine epizootic fever virus, Akabane virus, Japanese encephalitis virus, rabies virus and bovine RS virus showed good propagation.

Finally, for the attenuated bovine adeno 7 (AD7) virus, wild-type highly pathogenic Fukuroi strain was serially passaged in bovine kidney cell cultures, goat thymus cell cultures and goat testicle cell cultures at 30°C, after which cloning was done three times at 30°C, and the resulting TS-GT strain was used as the seed virus. This seed virus was inoculated into the goat testicle cell cultures at a m.o.i. of 0.1, and the virus fluid collected after culturing at 30 °C for 10-14 days was used as a virus suspension of the attenuated bovine adeno 7 (AD7) virus.

The virus content of each of these five types of virus fluid was adjusted to 10^{7.75}TCID₅₀/ml or more for the attenuated infectious bovine rhinotracheitis (IBR) virus fluid, 10^{6.00}TCID₅₀/ml or more for the attenuated bovine viral diarrhea-mucosal disease (BVD-MD) virus fluid, 10^{8.00}TCID₅₀/ml or more for the attenuated parainfluenza 3 (PI3) virus fluid, 10^{7.75}TCID₅₀/ml or more for the attenuated bovine RS virus fluid, and 10^{6.75}TCID₅₀/ml or more for the attenuated bovine adeno 7 (AD7) virus fluid.

To this virus stock fluid was added an equal amount of a stabilizer (an aqueous solution containing 5% by weight JIS reagent special grade lactose, 5% by weight JIS reagent special grade sucrose, 2% by weight JIS reagent special grade L-arginine hydrochloride, 3% by weight JIS reagent special grade glycine, 0.3% by weight JIS reagent special grade polyvinyl pyrrolidone, 0.5% by weight JIS reagent special grade lactalbumin and 0.1% by weight JIS reagent special grade yeast extract). The mixture was divided into vials,and then subjected to lyophilization.

Test results of vaccine dried through the above mentioned lyophilization and dissolved in a pre-prepared diluent were as follows. Test proving no abnormal toxicity:

An inoculation of the vaccine was made of 0.5 ml intraperitoneally in a 4-week-old mouse, and 5 ml intraperitoneally of a guinea pig, and both animals were observed for 10 days to detect any clinical symptoms or changes in body weight. The results are listed in Table 1.

As the results from these tests clearly show, no abnormalities were observed in the general clinical symptoms due to vaccination in both animal species, and the increase in body weight was satisfactory.

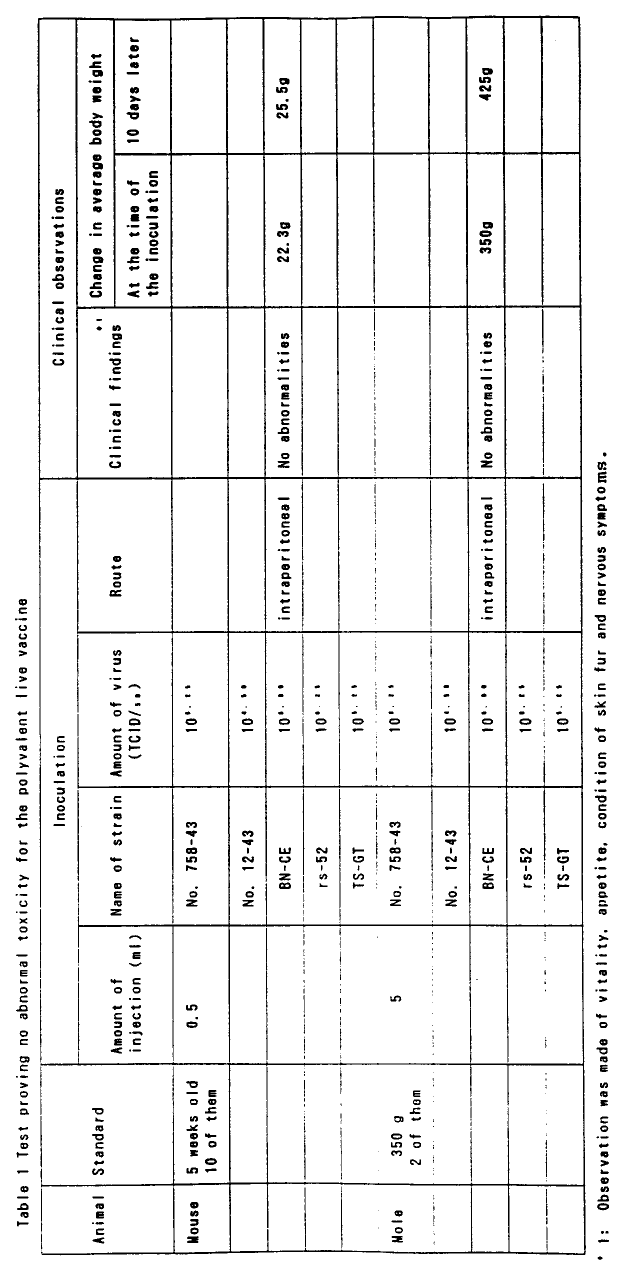

### Safety test in cattle:

Nine of 3 or 4-month-old Holstein cattle were used, of which three were given intramuscularly 1 dose each one time, three were given 100 doses each one time, while the remaining three were a non-inoculated control group; the general clinical symptoms were observed for 2 weeks. The results are listed in Table 2.

This data shows that there were no abnormalities observed regarding depression, anorexia, etc. for herds of cattle inoculated with 1 dose and 100 doses of the vaccine, respectively. Further, there was neither pyrexia (40.5°C or more) nor a leukopenia. Also, no swelling or induration was noticed at the locations of injection, nor were any side effects observed due to injection of the vaccine.

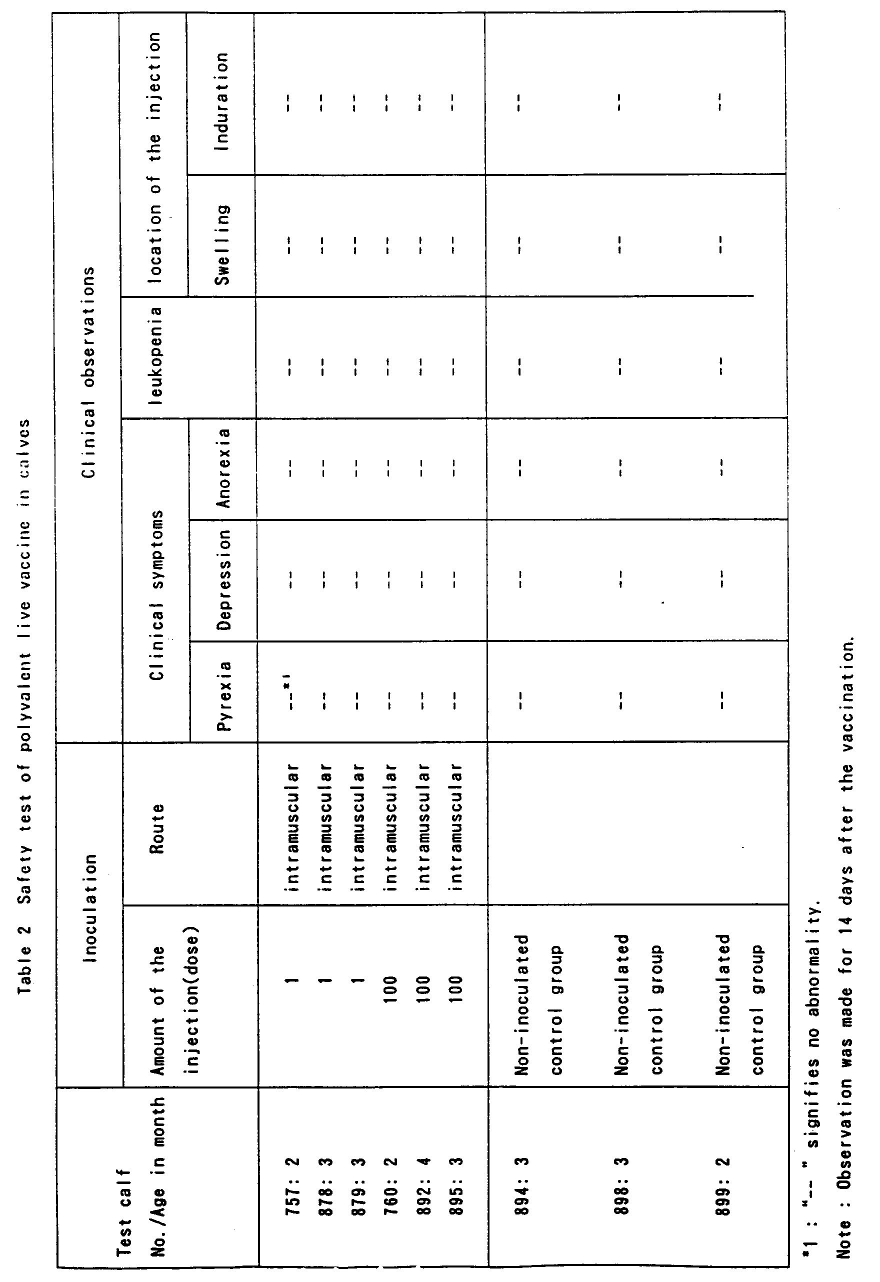

### Test of effectiveness (1):

Serum was obtained from the 9 cattle used in Table 2 prior to inoculation and at one week intervals until 4 weeks after inoculation, in order to measure the antibody response after inoculation, and the antibody titers were determined by using parental strains (wild type pathogenic strains) of each of the vaccine strains. The results are listed in Table 3.

The results showed that in cattle which were antibody negative before incubation, responses were confirmed 1-2 weeks after inoculation, for all the herds of cattle inoculated with either 1 dose or 100 doses of the vaccine, respectively. Even in some cattle, which were antibody positive before inoculation, a significant increase in antibody titer was observed.

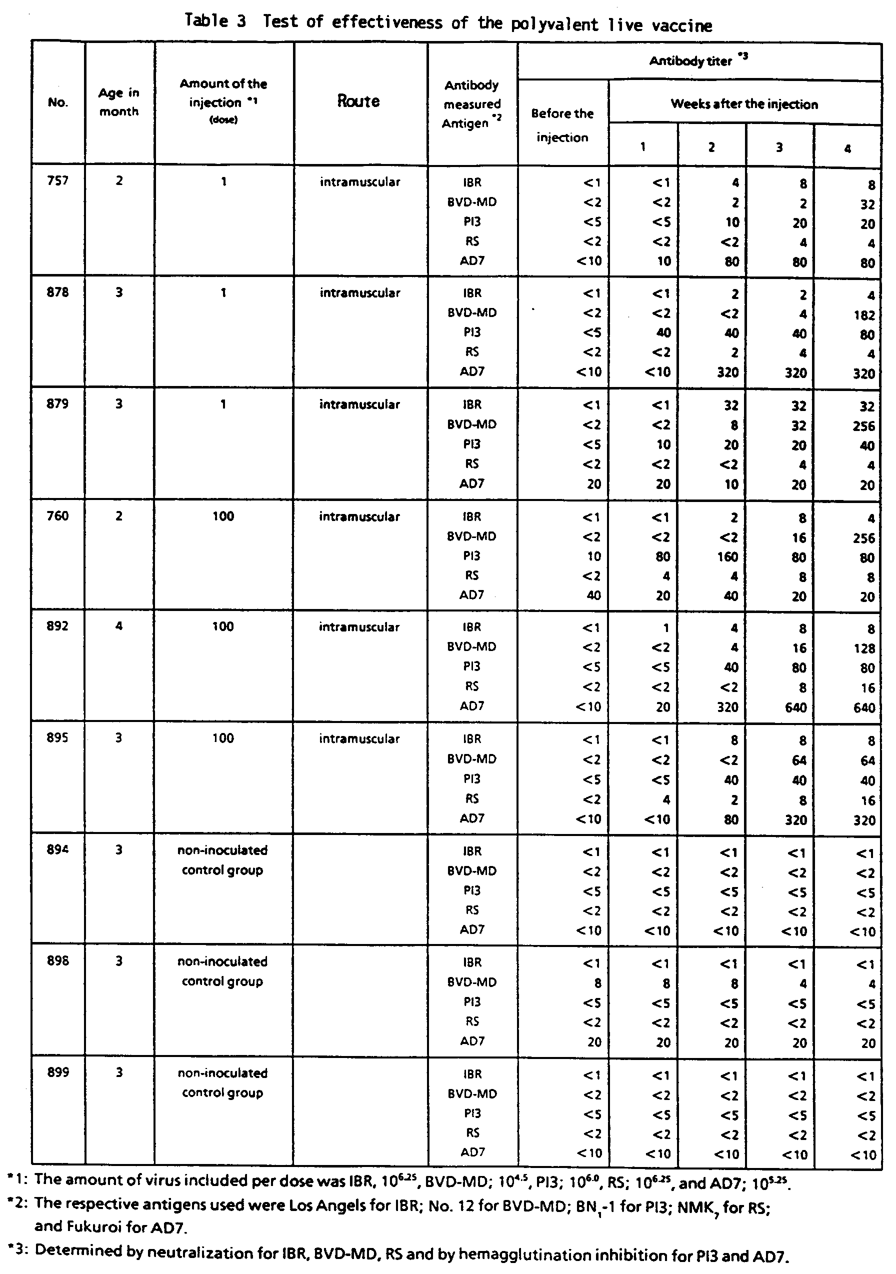

### Test of effectiveness (2):

Eighty field raised cattle were used, of which 60 were given 1 dose of the vaccine injected intramuscularly, while the remaining 20 were left as a non-inoculated control group. The antibody response in the vaccinated cattle was measured for up to 10 months after the vaccination, and titers were determined by using parental strains (wild type highly pathogenic) of each of the vaccine strains. The results are listed in Table 4.

**Table 4**

| Test of effectiveness of the polyvalent live vaccine (2) | | | | |
|---|---|---|---|---|
| Virus | Detected antibody | Rate of effectiveness one month after the vaccination (%) | | |
| | | Rate of positive conversion to antibody*¹ | Rate of significant increase in antibody titer*² | Rate of overall effectiveness *³ |
| IBR | NT*⁴ | 40/45*⁵(88.9) | 6/14 (42.9) | 46/59 (78.0) |
| BVD-MD | NT | 34/36 (94.4) | 11/23 (47.8) | 45/59 (76.3) |
| P13 | HI*⁶ | 34/39 (87.1) | 4/20 (20.0) | 38/59 (64.4) |
| RS | NT | 41/44 (93.2) | 3/15 (20.0) | 44/59 (74.6) |
| AD7 | HI | 38/38 (100) | 11/21 (52.4) | 49/59 (83.1) |

| | | | | |
|---|---|---|---|---|
| *1: Percentage of individual without antibodies at the time of the vaccination in which an antibody response was observed after the vaccination. The positive conversion to antibody was determined based on a standard neutralizing antibody titer of 1:2 or over for IBR, BVD-MD and RS and on a hemagglutination inhibition (HI) of 1:5 or over and 1:10 or over for P13 and AD, respectively. | | | | |
| *2: Percentage of individuals with antibodies at the time of the vaccination which exhibited an antibody titer after the vaccination of 4 times or over. | | | | |
| *3: Total percentage of individuals which showed a positive conversion to antibody and a significant increase in antibody titer. | | | | |
| *4: Neutralizing antibody. | | | | |
| *5: Number of antibody positive cattle/number of the cattle tested (%). | | | | |
| *6: Hemagglutination inhibition antibody. | | | | |

The antibody response of those cattle without antibodies before vaccination was tested using serum collected one month after vaccination, and showed a favorable positive conversion for the infectious bovine rhinotracheitis (IBR) virus for 40 of the 45 cattle (40/45=88.9%); the bovine viral diarrhea-mucosal disease (BVD-MD) virus (34/36=94.4%); the parainfluenza 3 (PI3) virus(34/39=87.1%); the bovine RS virus (41/44=93.2%); and the bovine adeno 7 (AD7) virus (38/38=100%).

The effectiveness of the developed stabilizer was compared with the known stabilizer. The results is given below.

### Test of effectiveness of stabilizer

Decrease rate of virus content in a polyvalent live vaccine of the present invention was examined in both cases, just after production and after 21 months' preservation at 4°C with stabilizer added thereto. Table 5 shows the results.

**Table 5**

| Effectiveness of stabilizer for preservation of virus | | | | |
|---|---|---|---|---|
| Stabilizer | Examined virus | Virus content ²⁾ | | Decrease rate after 21 months' preservation (%) ¹⁾ |
| | | Immediately after preparation | 21 months later (4 °C | |
| Developed stabilizer | IBR | 6.25 | 6.00 | 44 |
| | BVD-MD | 4.75 | 4.25 | 68 |
| | PI3 | 6.25 | 5.75 | 68 |
| | R S | 6.00 | 5.75 | 44 |
| | AD7 | 5.25 | 5.25 | 0 |
| Known stabilizer ³⁾ | IBR | 6.25 | 6.00 | 44 |
| | BVD-MD | 4.50 | 4.25 | 44 |
| | PI3 | 6.25 | 5.50 | 82 |
| | R S | 5.75 | 4.75 | 90 |
| | AD7 | 5.25 | 4.50 | 82 |

| | | | | |
|---|---|---|---|---|
| 1) Calculation was made on the basis of the virus content immediately after preparation. | | | | |
| 2) Virus content (log₁₀ TCID₅₀/dose) | | | | |
| 3) Purified water including 10% of lactose by The Pharmacopoeia of Japan and 0.3% of polyvinylpyrrolidone by The Pharmacopoeia of Japan. | | | | |

As it can be seen from the results in Table 5, the developed stabilizer is excellent compared with the known stabilizer in its property to stabilize each virus in vaccine. Specifically, in case where the known stabilizer was added, decrease rate of RS virus was 90% and that of AD7 virus was 82%. In contrast, the developed stabilizer provided an excellent result, that is, the decrease rate of RS virus and AD7 virus are 44% and 0% respectively.

## Claims

1. A polyvalent, attenuated live vaccine composed of a mixture of 5 types of virus suspensions, including a virus suspension prepared by culturing attenuated infectious bovine rhinotracheitis (IBP) virus in bovine or swine tissue cultures; a virus suspension prepared by culturing attenuated bovine viral diarrhea-mucosal disease (BVD-MD) virus in bovine or swine tissue cultures; a virus suspension prepared by culturing attenuated parainfluenza 3(PI3) virus in chicken or bovine tissue cultures, or monkey kidney (vero) cells; a virus suspension prepared by culturing attenuated bovine RS virus in hamster or bovine tissue cultures, or monkey kidney (vero) cells; and a virus suspension prepared by culturing attenuated bovine adeno 7 (AD7) virus in goat or bovine tissue cultures.

2. A polyvalent, attenuated live vaccine according to Claim 1, wherein attenuated infectious bovine rhinotracheitis (IBR) virus suspension is prepared by inoculation of an attenuated infectious bovine rhinotracheitis (IBR) virus strain into swine testicle cell cultures at a multiplicity of infection (amount of inoculated virus/number of cells grown) of 0.001, and culturing it.

3. A polyvalent, attenuated live vaccine according to Claim 1, wherein a bovine viral diarrhea-mucosal disease (BVD-MD) virus suspension is prepared by mixing an attenuated bovine viral diarrhea-mucosal disease (BVD-MD) virus strain into a suspension of swine testicle cells for inoculation at a multiplicity of infection (amount of inoculated virus/number of cells grown) of 0.5-0.1, and culturing the mixture.

4. A polyvalent, attenuated live vaccine according to Claim 1, wherein a parainfluenza 3 (PI3) virus suspension is prepared by mixing an attenuated parainfluenza 3 (PI3) virus strain into a suspension of chicken embryo cells for inoculation at a multiplicity of infection (amount of inoculated virus/number of cells grown) of 0.01, and culturing the mixture.

5. A polyvalent, attenuated live vaccine according to Claim 1, wherein a bovine RS virus suspension is prepared by inoculation of an attenuated bovine RS virus strain into highly virus-sensitive HAL cells obtained by cloning HAL cells, at a multiplicity of infection (amount of inoculated virus/number of cells grown) of 0.01, and culturing it, and concentrating of the obtained virus fluid using fractional ultrafiltration.

6. A polyvalent, attenuated live vaccine according to Claim 1, wherein a bovine adeno 7 (AD7) virus suspension is prepared by inoculation of an attenuated bovine adeno 7 (AD7) virus strain into goat testicle cell cultures at a multiplicity of infection (amount of inoculated virus/number of cells grown) of 0.1, and culturing it.

7. A polyvalent, attenuated live vaccine according to Claim 1, wherein the mixture ratio of each of the vaccine viruses is adjusted to 10^{7.75}TCID₅₀ or over for the infectious bovine rhinotracheitis (IBR) virus; 10^{6.00}TCID₅₀ or over for the bovine viral diarrhea-mucosal disease (BVD-MD) virus; 10^{8.00}TCID₅₀ or over for the parainfluenza 3 (PI3) virus; 10^{7.75}TCID₅₀ or over for the bovine RS virus; and 10^{6.75}TCID₅₀ or over for the bovine adeno 7 (AD7) virus.

## Patentansprüche

1. Polyvalenter, attenuierter Lebendimpfstoff zusammengesetzt aus einem Gemisch von 5 Typen von Virussuspensionen, umfassend eine Virussuspension hergestellt durch Züchtung von attenuiertem, infektiösem Rinder-Rhinotracheitisvirus (IBR) in Rinder- oder Schweinegewebekulturen; eine Virussuspension hergestellt durch Züchtung von attenuiertem, die virale Rinder-Schleimhautdiarrhöe-Krankheit verursachendem Virus (BVD-MD) in Rinder- oder Schweinegewebekulturen; eine Virussuspension hergestellt durch Züchtung von attenuiertem Parainfluenza 3-Virus (PI3) in Hühner- oder Rindergewebekultur oder Affennierenzellen (Vero); eine Virussuspension hergestellt durch Züchtung von attenuiertem Rinder-RS-Virus in Hamster- oder Rindergewebekultur oder Affennierenzellen (Vero); und eine Virussuspension hergestellt durch Züchtung von attenuiertem Rinder Adeno 7 (AD7)-Virus in Ziegen- oder Rindergewebekultur.

2. Polyvalenter, attenuierter Lebendimpfstoff nach Anspruch 1, wobei die attenuierte infektiöse Rinderrhinotracheitis (IBR)-Virussuspension hergestellt ist durch Inoculieren eines attenuierten infektiösen Rinderrhinotracheitis-Virus (IBR)-Stamms in Schweinehodenzellkulturen bei einer Multiplizität der Infektion (Menge des inoculierten Virus/Anzahl der gewachsenen Zellen) von 0,001 und dessen Züchtung.

3. Polyvalenter, attenuierter Lebendimpfstoff nach Anspruch 1, wobei eine Suspension aus die virale Rinder-Schleimhautdiarrhöe-Krankheit verursachendem Virus (BVD-MD) hergestellt ist durch Mischen eines attenuierten, die virale Rinder-Schleimhautdiarrhöe-Krankheit verursachenden Virus (BVD-MD)-Stammes in eine Suspension von Schweinehodenzellen zur Inoculation bei einer Multiplizität der Infektion (Menge des inoculierten Virus/Anzahl der gewachsenen Zellen) von 0,5 bis 0,1 und Züchtung des Gemisches.

4. Polyvalenter, attenuierter Lebendimpfstoff nach Anspruch 1, wobei eine Suspension von Parainfluenza 3 (PI3)-Virus hergestellt ist durch Mischen eines attenuierten Parainfluenza 3 (PI3)-Virusstammes in eine Suspension aus Hühnerembryozellen zur Inoculation bei einer Multiplizität der Infektion (Menge des inoculierten Virus/Anzahl der gewachsenen Zellen) von 0,01 und Züchtung des Gemisches.

5. Polyvalenter, attenuierter Lebendimpfstoff nach Anspruch 1, wobei eine Suspension von Rinder RS-Virus hergestellt ist durch Inoculation eines attenuierten Rinder-RS-Virusstammes in hoch virussensitive HAL-Zellen, erhalten durch Clonieren von HAL-Zellen, bei einer Multiplizität der Infektion (Menge des inoculierten Virus/Anzahl der gewachsenen Zellen) von 0,01 und dessen Züchtung, und Konzentrieren der erhaltenen Virusflüssigkeit unter Verwendung von fraktionierte- Ultrafiltration.

6. Polyvalenter, attenuierter Lebendimpfstoff nach Anspruch 1, wobei eine Suspension aus Rinderadeno 7 (AD7)-Virus hergestellt ist durch Inoculation eines attenuierten Rinderadeno 7 (AD7)-Virusstammes in Ziegenhodenzellkulturen bei einer Multiplizität der Infektion (Menge des inoculierten Virus/Anzahl der gewachsenen Zellen) von 0,1 und dessen Züchtung.

7. Polyvalenter, attenuierter Lebendimpfstoff nach Anspruch 1, wobei das Mischungsverhältnis jedes Impfstoffvirus eingestellt ist auf 10^{7.75}TCID₅₀ oder höher für das infektiöse Rinderrhinotracheitis-Virus (IBR); 10^{6.00}TCID₅₀ oder höher für das die virale Rinder-Schleimhautdiarhöe-Krankheit verursachende Virus (BVD-MD), 10^{8.00}TCID₅₀ oder höher für das Parainfluenza 3-Virus (PI3); 10^{7.75}TCID₅₀ oder höher für das Rinder RS-Virus; und 10^{6.75}TCID₅₀ oder höher für das Rinderadeno 7-Virus (AD7).

## Revendications

1. Vaccin vivant polyvalent atténué, constitué d'un mélange de 5 types de suspension de virus, comprenant une suspension de virus préparée en cultivant le virus atténué de l'infection bovine rhinotrachéite (IBR) dans des cultures de tissus bovins ou porcins; une suspension virale préparée en cultivant le virus atténué de la maladie bovine virale diarrhéique-mucosique (BVD-MD) dans des cultures de tissus bovins ou porcins; une suspension virale préparée en cultivant le virus atténué du parainfluenza 3 (PI3) dans des cultures de tissus de poulet ou de bovin ou des cellules de rein de singe (vero); une suspension virale préparée en cultivant le virus bovin atténué de la maladie bovine RS dans des cultures de tissus de hamster ou de bovin, ou des cellules de rein de singe (vero) et une suspension virale préparée en cultivant le virus atténué de l'adéno 7 bovin (AD7) dans des cultures de tissus de chèvre ou de bovin.

2. Vaccin vivant polyvalent atténué selon la revendication 1, dans lequel la suspension de virus atténué de l'infection bovine rhinotrachéite (IBR) est préparée par inoculation d'une souche virale atténuée de l'infection bovine rhinotrachéite (IBR) dans des cultures de cellules de testicule de porc et avec une multiplicité d'infections (quantité de virus inoculée/nombre de cellules cultivées) égale à 0,001 et en le cultivant.

3. Vaccin vivant polyvalent atténué selon la revendication 1, dans lequel la suspension virale de la maladie bovine diarrhéique-mucosique (BVD-MD) est préparée en melangeant une souche virale atténuée de la maladie bovine virale diarrhéique-mucosique (BVD-MD) à une suspension de cellules de testicules de porc pour l'inoculation avec un facteur d'infection (quantité de virus inoculée/nombre de cellules cultivées) compris etre 0,5 et 0,1 et en cultivant le mélange.

4. Vaccin vivant polyvalent atténué selon la revendication 1, dans lequel une suspension virale de parainfluenza 3 (PI3) est préparée en mélangeant une souche de virus atténuée de parainfluenza 3 (PI3) à une suspension de cellule d'embryon de poulet pour l'inoculation avec un facteur d'infection (quantité de virus inoculée/nombre de cellules cultivées) égal à 0,01, et en cultivant le mélange.

5. Vaccin vivant polyvalent atténué selon la revendication 1, dans lequel une suspension virale de la maladie bovine RS est préparée par inoculation d'une souche virale atténuée de la maladie bovine RS à des cellules HAL fortement sensibles au virus obtenues par clonage de cellules HAL avec un facteur d'infection (quantité de virus inoculée/nombre de cellules cultivées) égal à 0,01 puis en la cultivant et en concentrant le fluide viral obtenu en utilisant une ultrafiltration fractionnée.

6. Vaccin vivant polyvalent atténué selon la revendication 1, dans lequel une suspension virale bovine de l'adéno 7 (AD7) est préparée par inoculation d'une souche virale atténuée de la maladie bovine adéno 7 (AD7) dans des cultures de cellules de testicule de bouc avec un facteur d'infection (quantité de virus inoculée/nombre de cellules cultivées) égal à 0,1 puis en la cultivant.

7. Vaccin vivant polyvalent atténué selon la revendication 1, dans lequel le rapport de mélange de chacun des virus de vaccin est réglé à une valeur égale à 10^{7.75}TCID₅₀ ou davantage pour le virus de l'infection bovine rhinotrachéite (IBR); à une valeur égale à 10^{6.00}TCID₅₀ ou davantage pour le virus de la maladie bovine virale diarrhéique-mucosique (BVD-MD); à une valeur égale 10^{8.00}TCID₅₀ ou davantage pour le virus du parainfluenza 3 (PI3); à une valeur égale à 10^{7.75}TCID₅₀ ou davantage pour le virus bovin RS; et à une valeur égale à 10^{6.75}TCID₅₀ ou davantage pour l'adénovirus bovin 7 (AD7).
